# EUROPÄISCHE PATENTSCHRIFT

(11) **EP 1 614 409 B1**
(45) Veröffentlichungstag und Bekanntmachung des Hinweises auf die Patenterteilung: **16.08.2017**
(21) Anmeldenummer: 05013923.7
(22) Anmeldetag: 28.06.2005
(51) Int. Cl.: A61K 6/00, A61K 6/02, A61K 6/027

(54) **Opake Dentalkeramik, Verfahren zur Herstellung sowie deren Verwendung**
Opaque dental ceramic, process and use thereof
Céramique dentaire opaque, procédé et utilisation

(30) Priorität: 28.06.2004 EP 04015117
(43) Veröffentlichungstag der Anmeldung: 11.01.2006
(73) Patentinhaber: DENTSPLY International Inc., York PA 17401 (US)
(72) Erfinder: Krumbholz, Klaus., D-63225 Langen (DE)
(74) Vertreter: Stoffregen, Hans-Herbert

(56) Entgegenhaltungen:
- EP-A- 0 630 639
- EP-A- 1 452 500
- WO-A-2004/000743
- DE-A1- 3 840 195
- DE-A1- 19 852 516
- DE-C1- 4 423 793

## Beschreibung

Die Erfindung bezieht sich auf eine opake Dentalkeramik zum Aufbrennen auf ein Gerüst einer Zahnrestauration zumindest enthaltend SiO₂, Al₂O₃, B₂O₃, Na₂O, K₂O sowie TiO₂. Ferner bezieht sich die Erfindung auf ein Verfahren zum Herstellen einer opaken Dentalkeramik zum Aufbrennen auf ein Gerüst einer Zahnrestauration unter Verwendung einer Rohstoffmischung enthaltend SiO₂, AL₂O₃, B₂O₃, Na₂O, K₂O sowie TiO₂, Aufschmelzen der Rohstoffmischung, Fritten so hergestellter Glasschmelze, Mahlen und Überführen der Fritte in ein auf das Gerüst auftragfähiges Pulver.

Auch nimmt die Erfindung Bezug auf die Verwendung einer Fritte als opake Dentalkeramik zum Verblenden eines Gerüstes für eine Zahnrestauration.

Aus der DE-A-43 34 493 ist eine dentalkeramische Restauration mit einem mehrschichtigen Aufbau auf einem Kern bekannt, der erhältlich ist durch Aufbringen einer Opaker-Schicht der Zusammensetzung 50 bis 60 Gew.-% SiO₂, 4 bis 10 Gew.-% Na₂O, 0 bis 2,5 Gew.-% CaO, 8 bis 14 Gew.-% K₂O, 10 bis 20 Gew.-% Al₂O₃, 0 bis 6 Gew.-% B₂O₃, 5 bis 12 Gew.-% TiO₂ sowie Pigmenten, vorzugsweise auf Oxidbasis, auf einen Kern, gefolgt von Brennen dieser Anordnung.

In der DE-A-39 02 771 wird eine aufbrennbare silikatische Masse aus einer leicht fließenden Grundschmelze und einem Opaker-Anteil vorgeschlagen, die 38 bis 42 Gew.-% der gesamten Masse aufweist und 10,4 bis 11,5 Gew.-% TiO₂ enthält. Hierdurch bedingt sind Brenntemperatur und Wärmeausdehnungskoeffizient eingeschränkt mit der Folge, dass die silikatische Masse nur für die Verblendung einer begrenzten Anzahl von Legierungen von Bedeutung ist.

Als Hauptforderungen, die an opake Keramiken für die Verblendung von Zahnrestaurationen zu stellen sind, sind zu nennen:
- die Keramiken müssen opak genug sein, um das nicht zahnfarbene Gerüst durch Aufbrand einer möglichst nicht mehr als 0,2 mm dicken Schicht optisch abzudecken,
- die Aufbrenntemperatur muss auf die übrigen Materialien abgestimmt sein, wobei bei Aufbrand auf Legierungen die Aufbrenntemperatur vorzugsweise mindestens 100 °C unter der Solidustemperatur der Legierung liegen sollte,
- ein ausreichender Verbund mit dem Gerüst muss eingegangen werden. Dies setzt eine gute Benetzung des Grundes während des Aufbrennens und eine abgestimmte Wärmedehnung voraus.

In der Praxis werden üblicherweise Opakermassen wie folgt hergestellt. Es werden ein oder mehrere glasige oder leucithaltige Fritten erschmolzen. Nach Mahlung der Fritten werden diese mit Trübungsmitteln wie zum Beispiel Zinn-, Zirkon- oder Ceroxid vermischt, deren Anteil bis zu 30 Gew.-% betragen kann. Zur Erzielung einer homogenen Verteilung der Trübungsmittel und zur Verhinderung ihrer Separierung können diese Mischungen in einem zusätzlichen Arbeitsgang zusammengeschmolzen werden. Sodann wird der Schmelzkuchen fein aufgemahlen. Durch Zumischung geeigneter Pigmente können sodann die erforderlichen Farben eingestellt werden.

Nach den bekannten Verfahren ist es jedoch nicht möglich, das Potenzial der Trübungsmittel voll auszuschöpfen, da keine homogene Verteilung der trübenden Partikel erreicht wird. Vielmehr neigen die trübenden Partikel zur Konglomeration. Ferner ist es nach dem bekannten Verfahren nicht möglich, dass die Trübungsmittel eine optimale Partikelgröße erhalten, die im Wellenlängenbereich des Lichts, also unter 1 µm liegen sollte. Folglich ist eine unnötig große Menge an Trübungsmitteln erforderlich, ohne dass das erstrebenswerte Ziel erreicht wird, mit einer einzigen Opakerapplikation eine ausreichende Gerüstabdeckung zu erreichen. Ferner ist von Nachteil, dass die hochschmelzenden Oxide, die als Trübungsmittel benutzt werden, einen Beitrag zur Benetzung des zu verblendenden Gerüstmaterials beim Aufbrand nicht liefern. Infolgedessen kann eine ungenügende Haftung auftreten, die einen primären Brand mit einer besser benetzenden Keramik erforderlich macht.

Aus der DE-A-198 92 516 ist eine keramische Dentalrestauration bekannt, die als einzige Kristallphase Leucit mit einem Gesamtanteil von 20 bis 45 Gew.-% enthält. Als Komponenten sind zwingend vorgesehen 40 bis 95 Gew.-% SiO₂, 5 bis 25 Gew.-% Al₂O₃, 5 bis 25 Gew.-% K₂O. Ferner kann gewünschtenfalls TiO₂ und Na₂O vorliegen.

Eine opaleszierende Glaskeramik nach der WO-A-2004/000743 ist zwingend TiO₂ frei.

Nach der EP-A-0 622 342 enthält ein opaleszierendes Glas eine kontinuierliche Glasphase und eine darin verteilte diskontinuierliche Glasphase. Das Glas kann als Komponente TiO₂ in einer Menge aufweisen, in der es gelöst ist, um eine Trübung zu vermeiden, da anderenfalls die angestrebte Opaleszenz überdeckt wird.

In einer Dentalkeramik nach der US-A-2003/0122270 sind u. a. bis zu 2 Gew.-% TiO₂ sowie F vorhanden. Aufgrund der geringen Menge an TiO₂ bleibt dieses in Lösung. Durch den Bestandteil F kann die zur Herstellung der Keramik benötigte Schmelze dünnflüssiger gemacht werden. Nachteilig ist jedoch, dass bei der Herstellung Fluor frei wird.

In der DE-A-196 06 492 werden mit Kunststoffen überzogene Dentalprothesenteile sowie Verfahren zu ihrer Herstellung beschrieben. Dabei ist zwischen einem Gerüst und einer äußeren Kunststoffschicht eine Haftvermittlerschicht vorgesehen, die TiO₂ als Trübungsmittel enthalten kann.

Die ältere, jedoch nicht vorveröffentlichte EP-A-1 452 500 bezieht sich auf eine Glaskeramik sowie deren Herstellung. Die Glaskeramik weist eine Kristallphase aus Leucitkristallen auf, die homogen in einer Glasphase verteilt ist. Die Kristalle bestehen dabei aus Gruppen unterschiedlicher Korngrößen.

Aus der DE-C-44 23 793 ist eine leucithaltige Phosphorsilikat-Glaserkamik bekannt, die für Dentalprodukte verwendet werden. Die Glaskeramik soll nicht als Opaker verwendet werden, da eine Opaleszenz angestrebt wird. Dabei kann die Glaskeramik bis 3 Gew.-% TiO₂ enthalten.

Eine dentalkeramische Restauration mit einem mehrschichtigen Aufbau ist aus der EP-A-0 630 639 bekannt. Eine Opaker-Schicht als eine der Schichten enthält 5 - 12 Gew.-% TiO₂. Auf die Opaker-Schicht wird eine Dentin-Schmelzschicht aufgebracht.

Die DE-A-38 40 195 bezieht sich auf Titanoxid, das als Trübungsmittel für glasartige Emails und Schmelzglasglasur benutzt wird.

Der vorliegenden Erfindung liegt das Problem zu Grunde, eine opake Dentalkeramik, ein Verfahren zum Herstellen einer solchen sowie die Verwendung einer opaken Dentalkeramik derart weiterzubilden, dass im hinreichenden Umfang, ohne dass mehrere Opakerschichten aufgetragen werden müssen, eine hinreichende Abdeckung des nicht zahnfarbenen Gerüstmaterials erfolgt. Ferner soll eine optimale Partikelgröße der Trübungsmittel erzielt und ein Konglomerieren weitgehend vermieden werden. Auch soll eine gewünschte Anpassung an die physikalischen Eigenschaften des Grundgerüstes, insbesondere in Bezug auf die Wärmeausdehnung und Benetzung erfolgen.

Erfindungsgemäß wird das Problem durch eine opake Dentalkeramik der eingangs genannten Art im Wesentlichen dadurch gelöst, dass die opake Dentalkeramik durch Ausscheidung einer oder mehrerer kristalliner TiO₂-Phasen getrübt ist und Rutil und/oder Anatas als rekristallisiertes TiO₂ enthält, wobei 1 : 2 ≤ Anatas : Rutil ≤ 2 : 1 ist. Dabei erfolgt das Ausscheiden der kristallinen Phase/n durch Tempern des durch Fritten hergestellten Glases. Als Temperung ist nicht das Aufbrennen des Glaspulvers, also der gemahlenen Fritte auf das Gerüst zu verstehen.

Mit anderen Worten wird eine opake Dentalkeramik vorgeschlagen, die aufgrund einer Ausscheidungstrübung von zumindest einer kristallinen TiO₂-Phase (Anatas und/oder Rutil) eine so starke Opazität aufweist, dass bereits ein einmaliges Applizieren zum vollständigen Abdecken der Farbe des metallischen oder nichtmetallischen Gerüstes wie Kronen oder Brückengerüstes ausreicht. Die opake Dentalkeramik ist so eingefärbt, dass sie als Basis für die angestrebte Zahnfarbe dient. Dabei sollten die kristallinen TiO₂-Phasen mit färbenden Ionen wie Chrom-Ionen gesättigt bzw. in einem Umfang beladen sein, dass keine weiteren färbenden Ionen nach dem Auftragen auf ein Gerüst von der Keramik aufgenommen werden können und somit ein unerwünschtes Verfärben der fertigen Keramik (Verblendung) vermieden wird.

Insbesondere ist vorgesehen, dass die Dentalkeramik enthält

| | |
|---|---|
| SiO₂ | 44 - 54 Gew.-% |
| Al₂O₃ | 3 - 8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |

Alternativ ist vorgesehen:

| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,2 Gew.-% |

beziehungsweise

| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |

Die Gesamtmenge der Komponenten ergibt 100 Gew.-%.

Zur Erzielung eines hohen Wärmeausdehnungskoeffizienten kann die Dentalkeramik außerdem kristallines Leucit enthalten.

Unabhängig hiervon ist zur Erzielung einer gewünschten Färbung vorgesehen, dass im Rohmaterial der Dentalkeramik gemäß zuvor wiedergegebener Zusammensetzung/en Farboxide bis zu 0,2 Gew.-% zugegeben werden. Hierbei kann es sich um Oxide von Eisen, Chrom, Mangan und/oder Nickel handeln. Weitere Komponenten wie zum Beispiel ZnO, F, SnO₂, Sb₂O₃ und/oder CeO₂ können außerdem ohne Nachteile bis zu 2 Gew.-% zugegeben sein.

In der erfindungsgemäßen Dentalkeramik liegt eine kontinuierliche Glasphase mit zumindest einer darin diskontinuierlich verteilten Kristallphase (TiO₂) vor. Dabei ist die TiO₂-Kristallphase mit färbenden Ionen gesättigt bzw. in einem Umfang beladen, dass eine Nachverfärbung einer auf ein Gerüst aufgetragenen Dentalkeramik nicht erfolgen kann.

Ein Verfahren zum Herstellen einer opaken Dentalkeramik zum Aufbrennen auf ein Gerüst einer Zahnrestauration unter Verwendung einer Rohstoffmischung enthaltend SiO₂, Al₂O₃, B₂O₃, Na₂O, K₂O sowie TiO₂, Aufschmelzen der Rohstoffmischung, Fritten so hergestellter Glasschmelze, Mahlen und Überführen der Fritte in ein auf das Gerüst auftragfähiges Pulver ist dadurch gekennzeichnet, dass in dem durch Fritten hergestellten Glas enthaltenes TiO₂ durch Tempern des Glases rekristallisiert wird, wobei der Rohstoffmischung färbende Oxide in einem Umfang zugegeben werden, dass das rekristallisierte TiO₂ mit Rutil und/oder Anatas als rekristallisiertes TiO₂ mit 1 : 2 ≤ Anatas : Rutil ≤ 2 : 1 mit färbenden Ionen gesättigt oder weitgehend beladen ist, dass eine Verfärbung der auf das Gerüst aufgetragenen Keramik unterbleibt. Dabei wird das Glas bei einer Temperatur T mit 700 °C ≤ T ≤ 950 °C getempert. Das Tempern selbst kann über eine Zeit t mit 30 min. ≤ t ≤ 90 min. erfolgen.

Zur Herstellung der Dentalkeramik werden eine oder mehrere Fritten mit kristalliner TiO₂-Phase einer Rohstoffzusammensetzung verwendet

| | |
|---|---|
| SiO₂ | 44 - 54 Gew.-% |
| Al₂O₃ | 3-8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |

zur Erzeugung einer Fritte des Typs A und/oder

| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,2 Gew.-% |

zur Erzeugung einer Fritte des Typs B und/oder

| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |

zur Erzeugung einer Fritte des Typs C.

Insbesondere ist vorgesehen, dass dem Rohstoff zwischen 0 und 0,2 Gew.-% von einem oder mehreren Farboxiden in Form von Oxiden von Eisen, Chrom, Mangan und/oder Nickel zugegeben wird. Ferner können in dem Rohstoff als weitere Komponenten zum Beispiel ZnO, F, SnO₂, Sb₂O₃ und/oder CeO₂ bis zu 2 Gew.-% enthalten sein.

Zur Erzielung eines hohen Wärmeausdehnungskoeffizienten der opaken Dentalkeramik kann des Weiteren in der Glasschmelze Leucit kristallisiert werden.

Die Erfindung nimmt auch Bezug auf die Verwendung einer Fritte des Typs A mit einer Zusammensetzung

| | |
|---|---|
| SiO₂ | 4 - 54 Gew.-% |
| Al₂O₃ | 3 - 8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |

und/oder einer Fritte des Typs B mit einer Zusammensetzung

| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,2 Gew.-% |

und/oder einer Fritte des Typs C mit einer Zusammensetzung

| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |

wobei jede Fritte eine oder mehrere kristalline TiO₂-Phasen mit Rutil und/oder Anatas als rekristallisiertes Ti02 mit 1 : 2 ≤ Anatas : Rutil ≤ 2 : 1 enthält, als opake Dentalkeramik zum Verblenden eines Gerüstes für eine Zahnrestauration.

Die Gesamtmenge der Komponenten ergibt 100 Gew.-%.

Die Erfindung sieht ferner die Verwendung der Fritte des Typs A als Grundmasse zum Verblenden eines Gerüstes aus Titan oder einer Legierung mit Hauptbestandteil Titan vor.

Ferner wird die Verwendung der Fritte des Typs B als opake Grundmasse zum Verblenden eines oxidkeramischen Gerüstes vorgeschlagen.

Die Erfindung sieht auch eine Verwendung einer Fritte des Typs C mit kristallisiertem Leucit als Grundmasse für Aufbrennlegierungen vor.

Nach einem weiteren Vorschlag der Erfindung ist vorgesehen die Verwendung der Fritte des Typs C unter Verwendung der Fritte des Typs A und/oder des Typs B zum Verblenden eines Grundgerüsts einer Legierung mit einem Wärmeausdehnungskoeffizienten zwischen 12,5 und 13,5 x 10⁻⁶/K_{(20°C-400°C)}.

Die Fritte des Typs A wird vorzugsweise für Brenntemperaturen T_{B1} mit T_{B1} ≤ 820 °C und/oder einem Wärmeausdehnungskoeffizienten zwischen 7 und 9 x 10⁻⁶/K_{(20°C-400°C)} verwendet.

Die Fritte des Typs B ist insbesondere zur Verwendung zur Herstellung einer opaken Dentalkeramik mit einer Brenntemperatur T_{B2} mit T_{B2} ≥ 820 °C und/oder einem Wärmeausdehnungskoeffizienten zwischen 8 und 11 x 10⁻⁶/K_{(20°C-400°C)} bestimmt.

Schließlich ist die Fritte des Typs C mit Leucitausscheidung als opake Dentalkeramik mit einem Wärmeausdehnungskoeffizienten zwischen 12,0 und 16,5 x 10⁻⁶/K_{(20°C-400 °C)} geeignet.

Erfindungsgemäß wird eine opake Dentalkeramik vorgeschlagen, für die eine oder mehrere Fritten verwendet werden, deren Herstellung im Wesentlichen in folgenden Schritten besteht:
- Erschmelzen eines transparenten Glases aus einer TiO₂-haltigen Rohstoffmischung,
- Fritten der Glasschmelze
- Tempern des durch Fritten hergestellten Glases bei einer geeigneten Temperatur zum Zweck der Rekristallisation des TiO₂,
- Mahlung der teilkristallinen Glasfritte/n zur Überführung in ein auftragfähiges Pulver.

Je nach den geforderten Eigenschaften werden die Fritten allein oder in Abmischung mit anderen Fritten und/oder weiteren Trübungsmitteln und Pigmenten verwendet. Geringe Mengen in die Gläser eingeschmolzener Farboxide bewirken, dass diese bei der Kristallisation des TiO₂ in das Kristallgitter eingebunden werden und dieses im Hinblick auf die anzustrebende Zahnfarbe günstig färben.

Die Zusammensetzung der Gläser können in weiten Grenzen variiert werden, so dass ein breites Spektrum an Brenntemperaturen und Wärmedehnungen der Keramik erreicht werden kann. Zur Erzielung einer hohen Wärmedehnung (hoher Wärmeausdehnungskoeffizient) kann neben der bzw. den TiO₂-Phasen auch Leucit (K₂O·Al2O3·4SiO2) zur Kristallisation gebracht werden.

Erwähntermaßen sind Fritten vom Typ A insbesondere zum Verblenden von Titangerüsten geeignet, während Fritten vom Typ B zum Beispiel als opake Grundmasse für oxidkeramische Gerüste verwendet werden können. Fritten vom Typ C haben durch die Leucitkristallisation einen hohen Wärmeausdehnungskoeffizienten (WAK) und sind daher als Grundmasse für Aufbrennlegierungen geeignet. Bei relativ hohem K₂O-Gehalt erhält man Fritten mit einem WAK von über 14,0 x 10⁻⁶/K_{(20 °C - 400 °C}). Durch Abmischen einer entsprechenden Fritte des Typs C mit einer Fritte des Typs A und/oder des Typs B kann ein geeigneter WAK eingestellt werden, der im Hinblick auf marktübliche Legierungen zwischen 12,5 und 13,5 x 10⁻⁶/K_{(20 °C - 400 °C)} liegt. Bei relativ hohem Al₂O₃-Gehalt (größer als 16 Gew.-% bezogen auf die Zusammensetzung nach Abzug des TiO₂-Anteils) kann durch Reduzierung des K₂O-Gehalts aber auch eine Fritte erzeugt werden, die ohne Zumischung einer weiteren Fritte den erwünschten WAK aufweist.

Durch die optimale Ausnutzung des Trübungsmittels in Form der kristallinen TiO₂-Phase/n erfolgt eine optimale Abdeckung des metallischen oder nichtmetallischen Gerüstes, wobei durch Einfärben die Basis für die angestrebte Zahnfarbe erzielt wird.

Das Trübungsmittel TiO₂ wird dem Rohstoffgemisch (Rohgemenge) der Fritte zugemischt und löst sich im Glasfluss vollständig auf. Im nachfolgenden Temperprozess wird das Trübungsmittel in kristalliner Form wieder ausgeschieden und als Trübungsmittel wirksam. Durch Variation der Temperbedingungen (Temperatur und/oder Zeit) sowie durch geeignete Frittezusammensetzung lassen sich die ausgeschiedene Menge, die Modifikation und die Größe der Kristalle beeinflussen.

Dabei ist für die Rekristallisationstrübung TiO₂ besonderes geeignet, da es sich - zum Beispiel im Gegensatz zum SnO₂ - bei hohen Temperaturen in großen Mengen in der Glasschmelze löst und bei tieferen Temperaturen zum überwiegenden Teil wieder auskristallisiert wird. Die kristallinen TiO₂-Phasen haben außerdem von allen üblichen Trübungsmitteln den höchsten Brechungsindex (Rutil: n_{D} = 2,76; Anatas: n_{D} = 2,52) und somit die größte trübende Wirkung.

Rutil kristallisiert nadelig bis leistenförmig, während Anatas pyramidale Kristalle mit Korndurchmesssern unter 1 µm bildet.

Durch die Zusammensetzung der Fritte kann Einfluss darauf genommen werden, ob vorzugsweise Rutil oder Anatas kristallisiert.

Die Trübung durch Rekristallisation von TiO₂ ist zwar in der Emailletechnologie bekannt. Allerdings erfolgt das Rekristallisieren beim Aufbrennen des Emaillepulvers auf ein Blech. Im Gegensatz dazu wird die Rekristallisation erfindungsgemäß in einem gesonderten Verfahrensschritt, nämlich dem Tempern des aus der Rohstoffmischung hergestellten Glases durchgeführt. Würde man demgegenüber eine Rekristallisation beim Aufbrennen der Opakerschicht auf einem Gerüst einer Zahnrestauration durchführen, so würden sich keine optimalen Rekristallisationsraten ergeben, da die Aufbrenntemperatur nicht mit den für die Rekristallisation günstigen Temperaturen übereinstimmt. Ferner sind die Brennzeiten für eine maximale Ausscheidungsmenge zu kurz.

Es ist bekannt, dass eine unkontrollierte Aufnahme von färbenden Kationen, insbesondere von Chromionen im Kristallgitter des TiO₂ erfolgt, wobei eine ungewünschte Verfärbung des weißen TiO₂ in Richtung Ocker auftritt. Dabei kann Rutil mehr Farboxide aufnehmen als Anatas und ist daher intensiver gefärbt als Letzteres.

Bei der Herstellung dentaler Restaurationen kann dieses Problem gleichfalls entstehen, und zwar zum Beispiel durch die zu verblendende Legierung oder durch zugemischte Pigmente, die Farboxide enthalten. Um diese Nachteile zu vermeiden, ist erfindungsgemäß vorgesehen, dass gezielt Farboxide zum Rohgemenge zugegeben werden, so dass eine unkontrollierte Aufnahme von Fremdionen nicht zu einer unkontrollierten Verfärbung der Keramik führen kann. So kann zum Beispiel durch gezieltes Hinzufügen von Chrom-Eisen-Aluminium-Zink-Spinell, der als Braunfarbkörper im Handel erhältlich ist, in Mengen bis zu 0,2 % erreicht werden, dass eine gewünschte Ockerfarbe erzielbar ist. Hierdurch ergibt sich zudem der Vorteil, dass eine günstige Ausgangsbasis für die anzustrebende Zahnfarbe erreichbar ist im Vergleich zum Weiß des ungefärbten TiO₂. Ferner kann die Menge der zuzumischenden Pigmente, die bei dunklen Zahnfarben über 15 Gew.-% betragen kann, stark vermindert werden.

Es ist vorteilhaft, neben einer stark gefärbten Fritte auch eine helle Fritte zur Verfügung zu haben. So kann man durch Mischen beider Fritten und gegebenenfalls Hinzuziehung weiterer Pigmente die angestrebten Zahnfarben erreichen. Wie zuvor erläutert kann eine stark gefärbte Fritte am besten mit einer Rutilkristallisation erhalten werden, während man für eine Anataskristallisation sorgt, wenn eine helle Fritte erhalten werden soll. Vorteilhafterweise werden beim Tempern der Fritten die zur Erreichung der Zahnfarben notwendigen Pigmente mit eingefrittet. Stellt man zum Beispiel vier Opaker mit unterschiedlicher Färbung (zum Beispiel hellocker, rotocker, gelbocker, graubraun) her, so kann durch Mischen dieser Massen untereinander jede gewünschte Zahnfarbe erzielt werden.

Weitere Einzelheiten, Vorteile und Merkmale der Erfindung ergeben sich nicht nur aus den Ansprüchen, den diesen zu entnehmenden Merkmalen, sondern auch aus der nachfolgenden Beschreibung nachstehender bevorzugter Ausführungsbeispiele.

Der Tabelle I sind beispielhaft verschiedene Rohstoffzusammensetzungen von Fritten zu entnehmen.

| | Beispiel 1 | Beispiel 2 | Beispiel 3 | Beispiel 4 |
|---|---|---|---|---|
| SiO₂ | 50,7 | 47,7 | 42,2 | 38,2 |
| Al₂O₃ | 5,5 | 7,8 | 14,8 | 14,7 |
| B₂O₃ | 9,5 | 4,9 | 4,5 | 6,8 |
| P₂O₅ | 3,3 | 2,0 | | 1,8 |
| Li₂O | 1,9 | 0,7 | 0,8 | 0,9 |
| Na₂O | 5,4 | 8,6 | 9,3 | 9,8 |
| K₂O | 5,8 | 7,3 | 8,4 | 8,7 |
| CaO | 3,8 | 2,2 | 1,0 | 1,0 |
| BaO | 2,0 | | | |
| TiO₂ | 12,0 | 17,6 | 18,0 | 18,0 |
| CeO₂ | | 1,1 | | |
| MgO | | | 0,9 | |
| Farboxide | 0,1 | 0,1 | 0,1 | 0,1 |

| | | | | |
|---|---|---|---|---|
| *(Angaben in Gew.-%)* | | | | |

Das Beispiel 1 gehört zu Typ A zuvor erläuterter Fritten. Wie auch bei den übrigen Beispielen werden als Rohstoffe Kalifeldspat, Quarz, Borax, Titandioxid sowie Carbonate und Nitrate der Alkalien und Erdalkalien eingesetzt. Das Rohgemenge wird gut gemischt und in einem gasbeheizten Tropftiegelofen bei ca. 1.450 °C geschmolzen. Die Schmelze wird in einem Wasserbad aufgefangen. Die so erhaltene transparente Fritte wird getrocknet, grob gemahlen, auf Schamotteplatten aufgeschichtet und ca. 1 Stunde bei ca. 800 °C im Elektroofen getempert. Der zusammengesinterte Frittekuchen wird wiederum in Wasser abgeschreckt, getrocknet und zu feinem Pulver gemahlen, das nach Anmischen mit einer Flüssigkeit in dünner Schicht auf der vorgesehenen Unterlage applizierbar ist.

Die entsprechende Keramik ist opak und wegen fehlender Farboxide im Versatz nur schwach cremefarben. Die Kristallphase der Keramik besteht vorwiegend aus Anatas. Ihr Wärmeausdehnungskoeffizient beträgt 8,8 x 10⁻⁶/K_{(20 °C - 400 °C)}. Die Keramik kann beispielsweise bei 760 °C zum Beispiel auf ein Titangerüst aufgebrannt werden.

Die der Tabelle I zu entnehmende Zusammensetzung gemäß Beispiel 2 entspricht der Fritte des Typs B. Die Herstellung erfolgt entsprechend des Beispiels 1, wobei allerdings die Schmelztemperatur auf ca. 1.500 °C erhöht und die Temperung bei ca. 850 °C durchgeführt wird. Die so hergestellte Keramik ist opak und ockerfarben. Als Kristallphasen wurden Anatas und Rutil ermittelt. Die Aufbrenntemperatur beträgt ca. 860 °C und der WAK 9,8 x 10⁻⁶/K. Die Keramik kann zum Beispiel auf Zirkonoxidgerüste aufgebrannt werden.

Die Zusammensetzung gemäß Beispiel 3 entspricht der Fritte des Typs C. Die Herstellung erfolgt entsprechend der zuvor erläuterten Beispiele. Das Tempern wird bei einer Temperatur von ca. 900 °C durchgeführt. Als Kristallphasen treten Leucit und Anatas auf. Hierdurch ist die Fritte nur schwach cremefarben. Der WAK beträgt 12,7 x 10⁻⁶/K. Die Aufbrenntemperatur beträgt ca. 860 °C.

Die Zusammensetzung gemäß Beispiel 4 entspricht der Fritte des Typs C. Der Wärmeausdehnungskoeffizient liegt bei 12.7 x 10⁻⁶/K. Die Brenntemperatur beträgt ca. 860 °C. Die Fritte ist wegen der Rutilkristallisation und der Gegenwart von Farboxiden stark ockerfarben.

## Patentansprüche

1. Opake Dentalkeramik zum Aufbrennen auf ein Gerüst einer Zahnrestauration zumindest enthaltend SiO₂, Al₂O₃, B₂O₃, Na₂O, K₂O sowie TiO₂,
**dadurch gekennzeichnet,**
**dass** die opake Dentalkeramik durch Temperung erfolgte Ausscheidung einer oder mehrerer kristalliner TiO₂-Phasen getrübt ist und Rutil und/oder Anatas als rekristallisiertes TiO₂ enthält, wobei 1 : 2 ≤ Anatas : Rutil ≤ 2 : 1 ist.

2. Opake Dentalkeramik nach Anspruch 1,
**dadurch gekennzeichnet,**
**dass** die Dentalkeramik enthält
| | |
|---|---|
| SiO₂ | 44 - 54 Gew.-% |
| Al₂O₃ | 3 - 8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |

3. Opake Dentalkeramik nach Anspruch 1,
**dadurch gekennzeichnet,**
**dass** die Dentalkeramik enthält
| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,2 Gew.-% |

4. Opake Dentalkeramik nach Anspruch 1,
**dadurch gekennzeichnet,**
**dass** die Dentalkeramik enthält
| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |

5. Opake Dentalkeramik nach zumindest einem der vorhergehenden Ansprüche,
**dadurch gekennzeichnet,**
**dass** die Dentalkeramik kristallines Leucit enthält.

6. Opake Dentalkeramik nach zumindest einem der vorhergehenden Ansprüche,
**dadurch gekennzeichnet,**
**dass** die Dentalkeramik bis 0,2 Gew.-% von einem oder mehreren Farboxiden wie zum Beispiel Oxiden von Eisen, Chrom, Mangan und/oder Nickel enthält.

7. Opake Dentalkeramik nach zumindest einem der vorhergehenden Ansprüche,
**dadurch gekennzeichnet,**
**dass** die Dentalkeramik bis zu 2 Gew.-% einer oder mehrerer weiterer Komponenten wie ZnO, F, SnO₂, Sb₂O₃ und/oder CeO₂ enthält.

8. Verfahren zum Herstellen einer opaken Dentalkeramik zum Aufbrennen auf ein Gerüst einer Zahnrestauration unter Verwendung einer Rohstoffmischung enthaltend SiO₂, Al₂O₃, B₂O₃, Na₂O, K₂O sowie TiO₂, Aufschmelzen der Rohstoffmischung, Fritten so hergestellter Glasschmelze, Mahlen und Überführen der Fritte in ein auf das Gerüst auftragfähiges Pulver,
**dadurch gekennzeichnet,**
**dass** in dem durch Fritten hergestellten Glas enthaltenes TiO₂ durch Tempern des Glases rekristallisiert wird, wobei der Rohstoffmischung färbende Oxide in einem Umfang zugegeben werden, dass das rekristallisierte TiO₂ mit Rutil und/oder Anatas als rekristallisiertes TiO₂ mit 1 : 2 ≤ Anatas : Rutil ≤ 2 : 1 mit färbenden Ionen gesättigt oder weitgehend beladen ist, dass eine Verfärbung der auf das Gerüst aufgetragenen Keramik unterbleibt.

9. Verfahren nach Anspruch 8,
**dadurch gekennzeichnet,**
**dass** das Glas bei einer Temperatur T mit 700 °C ≤ T ≤ 950 °C getempert wird.

10. Verfahren nach Anspruch 8 oder 9,
**dadurch gekennzeichnet,**
**dass** das Tempern über eine Zeit t mit 30 min. ≤ t ≤ 90 min. erfolgt.

11. Verfahren nach zumindest einem der Ansprüche 8 bis 10,
**dadurch gekennzeichnet,**
**dass** zur Herstellung der Dentalkeramik eine oder mehrere Fritten mit kristalliner TiO₂-Phase einer Zusammensetzung verwendet wird
| | |
|---|---|
| SiO₂ | 44 - 54 Gew.-% |
| Al₂O₃ | 3 - 8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |
zur Erzeugung einer Fritte des Typs A und/oder
| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,3 Gew.-% |
zur Erzeugung einer Fritte des Typs B und/oder
| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |
zur Erzeugung einer Fritte des Typs C.

12. Verfahren nach zumindest einem der Ansprüche 8 bis 11,
**dadurch gekennzeichnet,**
**dass** dem Rohstoff bis zu 0,2 Gew.-% von einem oder mehreren Farboxiden in Form von Oxiden von Eisen, Chrom, Mangan und/oder Nickel zugegeben wird.

13. Verfahren nach zumindest einem der Ansprüche 8 bis 12,
**dadurch gekennzeichnet,**
**dass** dem Rohstoff als weitere Komponenten zum Beispiel ZnO, F, SnO₂, Sb₂O₃ und/oder CeO₂ bis zu 2 Gew.-% zugegeben wird.

14. Verfahren nach zumindest einem der Ansprüche 8 bis 13,
**dadurch gekennzeichnet,**
**dass** in der Glasschmelze Leucit kristallisiert wird.

15. Verwendung einer Fritte des Typs A mit der Zusammensetzung
| | |
|---|---|
| SiO₂ | 44 - 54 Gew.-% |
| Al₂O₃ | 3 - 8 Gew.-% |
| B₂O₃ | 8 - 12 Gew.-% |
| P₂O₅ | 0 - 4 Gew.-% |
| Li₂O | 0 - 3 Gew.-% |
| Na₂O | 5 - 9 Gew.-% |
| K₂O | 5 - 9 Gew.-% |
| MgO | 0 - 2 Gew.-% |
| CaO | 0 - 4 Gew.-% |
| BaO | 0 - 3 Gew.-% |
| TiO₂ | 8 - 16 Gew.-% |
| färbendes Oxid | < 0,2 Gew.-% |
und/oder einer Fritte des Typs B mit einer Zusammensetzung
| | |
|---|---|
| SiO₂ | 42 - 54 Gew.-% |
| Al₂O₃ | 5 - 10 Gew.-% |
| B₂O₃ | 2 - 10 Gew.-% |
| P₂O₅ | 0 - 4 Gew.-% |
| Li₂O | 0 - 2 Gew.-% |
| Na₂O | 4 - 11 Gew.-% |
| K₂O | 4 - 11 Gew.-% |
| MgO | 0 - 2 Gew.-% |
| CaO | 0 - 3 Gew.-% |
| BaO | 0 - 1 Gew.-% |
| TiO₂ | 12 - 20 Gew.-% |
| färbendes Oxid | < 0,2 Gew.-% |
und/oder einer Fritte des Typs C mit einer Zusammensetzung
| | |
|---|---|
| SiO₂ | 36 - 50 Gew.-% |
| Al₂O₃ | 12 - 16 Gew.-% |
| B₂O₃ | 2 - 8 Gew.-% |
| P₂O₅ | 0 - 3 Gew.-% |
| Li₂O | 0 - 2 Gew.-% |
| Na₂O | 5 - 11 Gew.-% |
| K₂O | 7 - 12 Gew.-% |
| MgO | 0 - 2 Gew.-% |
| CaO | 0 - 3 Gew.-% |
| BaO | 0 - 1 Gew.-% |
| TiO₂ | 15 - 20 Gew.-% |
| färbendes Oxid | < 0,2 Gew.-% |
wobei jede Fritte eine oder mehrere kristalline TiO₂-Phasen mit Rutil und/oder Anatas als rekristallisiertes TiO₂ mit 1 : 2 ≤ Anatas : Rutil ≤ 2 : 1 enthält, als opake Dentalkeramik zum Verblenden eines Gerüstes für eine Zahnrestauration.

16. Verwendung einer Fritte des Typs A gemäß Anspruch 15 als Grundmasse zum Verblenden eines Gerüstes aus Titan oder einer Legierung mit Hauptbestandteil Titan.

17. Verwendung einer Fritte des Typs B gemäß Anspruch 15 als opake Grundmasse zum Verblenden eines oxidkeramischen Gerüstes.

18. Verwendung einer Fritte des Typs C gemäß Anspruch 15 mit kristallisiertem Leucit als Grundmasse zum Verblenden für Aufbrennlegierungen.

19. Verwendung der Fritte des Typs C gemäß Anspruch 15 unter Verwendung der Fritte des Typs A gemäß Anspruch 15 und/oder des Typs B gemäß Anspruch 15 zum Verblenden eines Grundgerüsts einer Legierung mit einem Wärmeausdehnungskoeffizienten zwischen 12,5 und 13,5 x 10⁻⁶/K_{(20°C-400°C)}.

## Claims

1. An opaque dental ceramic for burning on a framework of a dental restoration, the opaque dental ceramic at least comprising SiO₂, Al₂O₃, B₂O₃, Na₂O, K₂O as well as TiO₂, wherein the opaque dental ceramic is clouded through precipitation of one or more crystalline TiO₂ through tempering, and comprising rutile and/or anatase as recrystallized TiO₂, with 1 : 2 ≤ anatase : rutile ≤ 2 : 1.

2. The opaque dental ceramic according to claim 1, wherein the opaque dental ceramic comprises:
| | |
|---|---|
| SiO₂ | 44 - 54 weight % |
| Al₂O₃ | 3 - 8 weight % |
| B₂O₃ | 8 - 12 weight % |
| P₂O₅ | 0 - 4 weight % |
| Li₂O | 0 - 3 weight % |
| Na₂O | 5 - 9 weight % |
| K₂O | 5 - 9 weight % |
| MgO | 0 - 2 weight % |
| CaO | 0 - 4 weight % |
| BaO | 0 - 3 weight % |
| TiO₂ | 8 - 16 weight % |
| colouring oxide | < 0,2 weight % |

3. The opaque dental ceramic according to claim 1, wherein the dental ceramic comprises:
| | |
|---|---|
| SiO₂ | 42 - 54 weight % |
| Al₂O₃ | 5 - 10 weight % |
| B₂O₃ | 2 - 10 weight % |
| P₂O₅ | 0 - 4 weight % |
| Li₂O | 0 - 2 weight % |
| Na₂O | 4 - 11 weight % |
| K₂O | 4 - 11 weight % |
| MgO | 0 - 2 weight % |
| CaO | 0 - 3 weight % |
| BaO | 0 - 1 weight % |
| TiO₂ | 12 - 20 weight % |
| colouring oxide | < 0,2 Weight % |

4. The opaque dental ceramic according to claim 1, wherein the opaque dental ceramic comprises:
| | |
|---|---|
| SiO₂ | 36 - 50 weight % |
| Al₂O₃ | 12 - 16 weight % |
| B₂O₃ | 2 - 8 weight % |
| P₂O₅ | 0 - 3 weight % |
| Li₂O | 0 - 2 weight % |
| Na₂O | 5 - 11 weight % |
| K₂O | 7 - 12 weight % |
| MgO | 0 - 2 weight % |
| CaO | 0 - 3 weight % |
| BaO | 0 - 1 weight % |
| TiO₂ | 15 - 20 weight % |
| colouring oxide | < 0,2 weight % |

5. The dental ceramic according to at least one of the preceding claims, wherein the opaque dental ceramic contains crystalline leucite.

6. The opaque dental ceramic according to at least one of the preceding claims, wherein the opaque dental ceramic contains up to 0.2 weight % of one or more coloring oxides such as for example oxides comprising iron, chrome, manganese and/or nickel.

7. The opaque dental ceramic according to at least one of the preceding claims, wherein the opaque dental ceramic contains up to 2 weight % of one or more further components such as ZnO, F, SnO₂, Sb₂O₃ and/or CeO₂.

8. A process for the production of an opaque dental ceramic to burn on a framework of a dental restoration using a composition of raw materials comprising SiO₂, Al₂O₃, B₂O₃, Na₂O, K₂O as well as TiO₂, melting of the composition of raw materials, fritting of so produced glass, grinding and transfer of the frit in a powder applicable to the framework, wherein the glass produced in a frit comprises TiO₂ recrystallized by tempering of the glass, wherein coloring oxides are added to the composition of raw materials so that the recrystallized TiO₂, with rutile and/or anatase as recrystallized TiO₂ with 1 : 2 ≤ anatase : rutile ≤ 2 : 1, is largely loaded or saturated with colored ions so that a discoloration of the ceramic applied to the framework is avoided.

9. The process according to claim 8, wherein tempering of the glass takes place at a temperature of 700 °C ≤ T ≤ 950 °C.

10. The process according to claim 8 or 9, wherein the tempering takes place over a time t of 30 min. ≤ t ≤ 90 min.

11. A process according to at least one of the claims 8 to 10, wherein for the production of the dental ceramic one or more frits with crystalline TiO₂ phase of the following composition are used:
| | |
|---|---|
| SiO2 | 44 - 54 weight % |
| Al2O3 | 3 - 8 weight % |
| B2O3 | 8 - 12 weight % |
| P2O5 | 0 - 4 weight % |
| Li2O | 0 - 3 weight % |
| Na2O | 5 - 9 weight % |
| K2O | 5 - 9 weight % |
| MgO | 0 - 2 weight % |
| CaO | 0 - 4 weight % |
| BaO | 0 - 3 weight % |
| TiO2 | 8 - 16 weight % |
| colouring oxide | < 0,2 weight % |
for the production of a frit of type A and/or
| | |
|---|---|
| SiO2 | 42 - 54 weight % |
| Al2O3 | 5 - 10 weight % |
| B2O3 | 2 - 10 weight % |
| P2O5 | 0 - 4 weight % |
| Li2O | 0 - 2 weight % |
| Na2O | 4 - 11 weight % |
| K2O | 4 - 11 weight % |
| MgO | 0 - 2 weight % |
| CaO | 0 - 3 weight % |
| BaO | 0 - 1 weight % |
| TiO2 | 12 - 20 weight % |
| colouring oxide | < 0,3 weight % |
for the production of a frit of type B and/or
| | |
|---|---|
| SiO2 | 36 - 50 weight % |
| Al2O3 | 12 - 16 weight % |
| B2O3 | 2 - 8 weight % |
| P2O5 | 0 - 3 weight % |
| Li2O | 0 - 2 weight % |
| Na2O | 5 - 11 weight % |
| K2O | 7 - 12 weight % |
| MgO | 0 - 2 weight % |
| CaO | 0 - 3 weight % |
| BaO | 0 - 1 weight % |
| TiO2 | 15 - 20 weight % |
| colouring oxide | < 0,2 weight % |
for production of a frit of type C.

12. The process according to at least one of the claims 8 to 11, wherein up to 0.2 weight % of one or more coloring oxides in form of oxides comprising iron, chrome, manganese and/or nickel are added.

13. The process according to at least one of the claims 8 to 12, wherein up to 2 weight % of a further compound for example selected from the group comprising ZnO, F, SnO₂, Sb₂O₃ and/or CeO₂ are added.

14. The process according to at least one of the claims 8 to 13, wherein in the molten glass leucite is crystallized.

15. A use of a frit of type A with a composition:
| | |
|---|---|
| SiO2 | 44 - 54 weight % |
| Al2O3 | 3 - 8 weight % |
| B2O3 | 8 - 12 weight % |
| P2O5 | 0 - 4 weight % |
| Li2O | 0 - 3 weight % |
| Na2O | 5 - 9 weight % |
| K2O | 5 - 9 weight % |
| MgO | 0 - 2 weight % |
| CaO | 0 - 4 weight % |
| BaO | 0 - 3 weight % |
| TiO2 | 8 - 16 weight % |
| colouring oxide | < 0,2 weight % |
and/or a frit of type B with a composition;
| | |
|---|---|
| SiO₂ | 42 - 54 weight % |
| Al₂O₃ | 5 - 10 weight % |
| B₂O₃ | 2 - 10 weight % |
| P₂O₅ | 0 - 4 weight % |
| Li₂O | 0 - 2 weight % |
| Na₂O | 4 - 11 weight % |
| K₂O | 4 - 11 weight % |
| MgO | 0 - 2 weight % |
| CaO | 0 - 3 weight % |
| BaO | 0 - 1 weight % |
| TiO₂ | 12 - 20 weight % |
| colouring oxide | < 0,2 weight % |
and/or a frit of type C with a composition;
| | |
|---|---|
| SiO₂ | 36 - 50 weight |
| Al₂O₃ | 12 - 16 weight |
| B₂O₃ | 2 - 8 weight |
| P₂O₅ | 0 - 3 weight |
| Li₂O | 0 - 2 weight |
| Na₂O | 5 - 11 weight |
| K₂O | 7 - 12 weight |
| MgO | 0 - 2 weight |
| CaO | 0 - 3 weight |
| BaO | 0 - 1 weight |
| TiO₂ | 15 - 20 weight |
| colouring oxide | < 0,2 weight |
wherein each frit comprises one or more crystalline TiO₂ with rutile and/or anatase as recrystallized TiO₂, with 1 : 2 ≤ anatase : rutile ≤ 2 : 1, as opaque dental ceramic for a veneering of a framework of a dental restauration.

16. The use according to claim 15, wherein the frit is type A, and is a matrix for a veneering of a framework made from titanium or an alloy with titanium as primary component.

17. The use of according to claim 15, wherein the frit is type B, and is an opaque matrix for a veneering of an oxide ceramic framework.

18. The use of according to claim 15, wherein the frit is type C with crystallized leucite and is a matrix for a veneering for an alloy suitable for burning on.

19. The use of according to claim 15, wherein the frit is type C with use of the frit of type A according to claim 15 and/or the frit of B according to claim 15 and is a veneering for a framework of an alloy having a coefficient of thermal expansion between 12.5 and 13.5 x 10⁻⁶/K_{(20°C-400°C).}

## Revendications

1. Céramique dentaire opaque pour cuisson sur une armature d'une restauration dentaire comprenant au moins les composants suivants : SiO₂, Al₂O₃, B₂O₃, Na₂O, K₂O et TiO₂,
**caractérisée en ce**
**que** la céramique dentaire opaque s'opacifie par précipitation, obtenue par trempe, d'une ou plusieurs phases cristallines de TiO₂ et contient du rutile et/ou de l'anatase sous forme de TiO₂ recristallisé, sachant que 1 : 2 ≤ anatase : rutile ≤ 2 : 1.

2. Céramique dentaire opaque selon la revendication 1,
**caractérisée en ce**
**que** la céramique dentaire contient
| | |
|---|---|
| SiO₂ | 44 - 54 % en poids |
| Al₂O₃ | 3 - 8 % en poids |
| B₂O₃ | 8 - 12 % en poids |
| P₂O₅ | 0 - 4 % en poids |
| Li₂O | 0 - 3 % en poids |
| Na₂O | 5 - 9 % en poids |
| K₂O | 5 - 9 % en poids |
| MgO | 0 - 2 % en poids |
| CaO | 0 - 4 % en poids |
| BaO | 0 - 3 % en poids |
| TiO₂ | 8 - 16 % en poids |
| Oxyde colorant | < 0,2 % en poids |

3. Céramique dentaire opaque selon la revendication 1,
**caractérisée en ce**
**que** la céramique dentaire contient
| | |
|---|---|
| SiO₂ | 42 - 54 % en poids |
| Al₂O₃ | 5 - 10 % en poids |
| B₂O₃ | 2 - 10 % en poids |
| P₂O₅ | 0 - 4 % en poids |
| Li₂O | 0 - 2 % en poids |
| Na₂O | 4 - 11 % en poids |
| K₂O | 4 - 11 % en poids |
| MgO | 0 - 2 % en poids |
| CaO | 0 - 3 % en poids |
| BaO | 0 - 1 % en poids |
| TiO₂ | 12 - 20 % en poids |
| Oxyde colorant | < 0,2 % en poids |

4. Céramique dentaire opaque selon la revendication 1,
**caractérisée en ce**
**que** la céramique dentaire contient
| | |
|---|---|
| SiO₂ | 36 - 50 % en poids |
| Al₂O₃ | 12 - 16 % en poids |
| B₂O₃ | 2 - 8 % en poids |
| P₂O₅ | 0 - 3 % en poids |
| Li₂O | 0 - 2 % en poids |
| Na₂O | 5 - 11 % en poids |
| K₂O | 7 - 12 % en poids |
| MgO | 0 - 2 % en poids |
| CaO | 0 - 3 % en poids |
| BaO | 0 - 1 % en poids |
| TiO₂ | 15 - 20 % en poids |
| Oxyde colorant | < 0,2 % en poids |

5. Céramique dentaire opaque selon au moins l'une des revendications précédentes,
**caractérisée en ce**
**que** la céramique dentaire contient de la leucite cristalline.

6. Céramique dentaire opaque selon au moins l'une des revendications précédentes,
**caractérisée en ce**
**que** la céramique dentaire contient jusqu'à 0,2 % en poids d'un ou de plusieurs oxydes colorants tels que l'oxyde de fer, de chrome, de manganèse et/ou de nickel.

7. Céramique dentaire opaque selon au moins l'une des revendications précédentes,
**caractérisée en ce**
**que** la céramique dentaire contient jusqu'à 2 % en poids d'un ou de plusieurs autres composants tels que ZnO, F, SnO₂, Sb₂ O₃ et/ou CeO₂.

8. Procédé de fabrication d'une céramique dentaire opaque pour cuisson sur une armature d'une restauration dentaire en utilisant un mélange de matières premières comprenant les composants suivants : SiO₂, Al₂O₃, B₂O₃, Na₂O, K₂O et TiO₂, fusion du mélange de matières premières, vitrification du bain de verre ainsi obtenu, mouture et transformation de la fritte en une poudre pouvant être appliquée sur l'armature,
**caractérisé en ce**
**que** le TiO₂ contenu dans le verre fabriqué par vitrification est recristallisé par trempe du verre, sachant que des oxydes colorants sont ajoutés au mélange de matières premières dans une quantité telle que le TiO₂ recristallisé avec du rutile et/ou de l'anatase est saturé ou largement chargé en ions colorants en tant que TiO₂ recristallisé à 1 : 2 ≤ anatase : rutile ≤ 2 : 1 et qu'une coloration de la céramique appliquée sur l'armature n'a pas lieu.

9. Procédé selon la revendication 8,
**caractérisé en ce**
**que** le verre est trempé à une température T telle que 700 °C ≤ T ≤ 950 °C.

10. Procédé selon la revendication 8 ou 9,
**caractérisé en ce**
**que** la trempe a lieu sur une période t telle que 30 min. ≤ t ≤ 90 min.

11. Procédé selon au moins l'une des revendications 8 à 10,
**caractérisé en ce**
**que**, pour fabriquer la céramique dentaire, est/sont utilisée(s) une ou plusieurs frittes de phase cristalline de TiO₂ d'une composition
| | |
|---|---|
| SiO₂ | 44 - 54 % en poids |
| Al₂O₃ | 3 - 8 % en poids |
| B₂O₃ | 8 - 12 % en poids |
| P₂O₅ | 0 - 4 % en poids |
| Li₂O | 0 - 3 % en poids |
| Na₂O | 5 - 9 % en poids |
| K₂O | 5 - 9 % en poids |
| MgO | 0 - 2 % en poids |
| CaO | 0 - 4 % en poids |
| BaO | 0 - 3 % en poids |
| TiO₂ | 8 - 16 % en poids |
| Oxyde colorant | < 0,2 % en poids |
pour obtenir une fritte du type A et/ou
| | |
|---|---|
| SiO₂ | 42 - 54 % en poids |
| Al₂O₃ | 5 - 10 % en poids |
| B₂O₃ | 2 - 10 % en poids |
| P₂O₅ | 0 - 4 % en poids |
| Li₂O | 0 - 2 % en poids |
| Na₂O | 4 - 11 % en poids |
| K₂O | 4 - 11 % en poids |
| MgO | 0 - 2 % en poids |
| CaO | 0 - 3 % en poids |
| BaO | 0 - 1 % en poids |
| TiO₂ | 12 - 20 % en poids |
| Oxyde colorant | < 0,3 % en poids |
pour obtenir une fritte du type B et/ou
| | |
|---|---|
| SiO₂ | 36 - 50 % en poids |
| Al₂O₃ | 12 - 16 % en poids |
| B₂O₃ | 2 - 8 % en poids |
| P₂O₅ | 0 - 3 % en poids |
| Li₂O | 0 - 2 % en poids |
| Na₂O | 5 - 11 % en poids |
| K₂O | 7 - 12 % en poids |
| MgO | 0 - 2 % en poids |
| CaO | 0 - 3 % en poids |
| BaO | 0 - 1 % en poids |
| TiO₂ | 15 - 20 % en poids |
| Oxyde colorant | < 0,2 % en poids |
pour obtenir une fritte du type C.

12. Procédé selon au moins l'une des revendications 8 à 11,
**caractérisé en ce**
**qu'**est ajouté à la matière première jusqu'à 0,2 % en poids d'un ou plusieurs oxydes colorants sous forme d'oxyde de fer, de chrome, de manganèse et/ou de nickel.

13. Procédé selon au moins l'une des revendications 8 à 12,
**caractérisé en ce**
**que** sont ajoutés à la matière première d'autres composants tels que ZnO, F, SnO₂, Sb₂O₃ et/ou CeO₂ jusqu'à 2 % en poids

14. Procédé selon au moins l'une des revendications 8 à 13,
**caractérisé en ce**
**que** de la leucite est cristallisée dans le bain de verre.

15. Utilisation d'une fritte du type A présentant la composition
| | | |
|---|---|---|
| SiO₂ | 44 - 54 | % en poids |
| Al₂O₃ | 3 - 8 | % en poids |
| B₂O₃ | 8 - 12 | % en poids |
| P₂O₅ | 0 - 4 | % en poids |
| Li₂O | 0 - 3 | % en poids |
| Na₂O | 5 - 9 | % en poids |
| K₂O | 5 - 9 | % en poids |
| MgO | 0 - 2 | % en poids |
| CaO | 0 - 4 | % en poids |
| BaO | 0 - 3 | % en poids |
| TiO₂ | 8 - 16 | % en poids |
| Oxyde colorant | < 0,2 | % en poids |
et/ou d'une fritte du type B présentant une composition
| | | |
|---|---|---|
| SiO₂ | 42 - 54 | % en poids |
| Al₂O₃ | 5 - 10 | % en poids |
| B₂O₃ | 2 - 10 | % en poids |
| P₂O₅ | 0 - 4 | % en poids |
| Li₂O | 0 - 2 | % en poids |
| Na₂O | 4 - 11 | % en poids |
| K₂O | 4 - 11 | % en poids |
| MgO | 0 - 2 | % en poids |
| CaO | 0 - 3 | % en poids |
| BaO | 0 - 1 | % en poids |
| TiO₂ | 12 - 20 | % en poids |
| Oxyde colorant | < 0,2 | % en poids |
et/ou d'une fritte du type C présentant une composition
| | | |
|---|---|---|
| SiO₂ | 36 - 50 | % en poids |
| Al₂O₃ | 12 - 16 | % en poids |
| B₂O₃ | 2 - 8 | % en poids |
| P₂O₅ | 0 - 3 | % en poids |
| Li₂O | 0 - 2 | % en poids |
| Na₂O | 5 - 11 | % en poids |
| K₂O | 7 - 12 | % en poids |
| MgO | 0 - 2 | % en poids |
| CaO | 0 - 3 | % en poids |
| BaO | 0 - 1 | % en poids |
| TiO₂ | 15 - 20 | % en poids |
| Oxyde colorant | < 0,2 | % en poids |
sachant que chaque fritte contient une ou plusieurs phases cristallines de TiO₂ avec du rutile et/ou de l'anatase sous forme de TiO₂ recristallisé avec 1 : 2 ≤ anatase : rutile ≤ 2 : 1, comme céramique dentaire opaque destinée à revêtir une armature pour une restauration dentaire.

16. Utilisation d'une fritte du type A selon la revendication 15 comme masse de base pour revêtir une armature en titane ou en alliage composé principalement de titane.

17. Utilisation d'une fritte du type B selon la revendication 15 comme masse de base opaque pour revêtir une armature en céramique oxydée.

18. Utilisation d'une fritte du type C selon la revendication 15 avec leucite cristallisée comme masse de base de revêtement pour des alliages céramisables.

19. Utilisation de la fritte du type C selon la revendication 15 avec utilisation de la fritte du type A selon la revendication 15 et/ou du type B selon la revendication 15 pour revêtir une armature de base d'un alliage présentant un coefficient de dilatation thermique compris entre 12,5 et 13,5 x 10⁻⁶/K_{(20 °C-400 °C)}.
